# EUROPEAN PATENT SPECIFICATION

(11) **EP 2 305 118 B1**
(45) Date of publication and mention of the grant of the patent: **04.01.2017**
(21) Application number: 09766565.7
(22) Date of filing: 10.06.2009
(51) Int. Cl.: A61B 5/22, A61B 5/0488, A61B 5/11

(54) **MUSCLE TONE MEASURING APPARATUS**
GERÄT ZUR MESSUNG DES MUSKELTONUS
APPAREIL DE MESURE DE TONICITÉ MUSCULAIRE

(30) Priority: 20.06.2008 JP 2008162048
(43) Date of publication of application: 06.04.2011
(73) Proprietor: Osaka University, Suita-shi Osaka 565-0871 (JP)
(72) Inventor: SAKODA, Saburo, Osaka 565-0871 (JP); AKAZAWA, Kenzo, Osaka 535-8585 (JP); OKUNO, Ryuhei, Osaka 565-0871 (JP); YOKOE, Masaru, Osaka 565-0871 (JP); ENDO, Takuyuki, Osaka 565-0871 (JP)
(74) Representative: Lees, Gregory Alexander
(86) International application number: PCT/JP2009/060615
(87) International publication number: WO 2009/154117

(56) References cited:
- EP-A2- 0 310 901
- WO-A1-02/19907
- JP-A- 7 059 759
- JP-A- 10 149 445
- JP-A- 2001 054 507
- JP-A- 2006 014 837
- JP-A- 2007 061 121
- US-A- 4 664 130
- US-A- 4 667 513
- ARTHUR PROCHAZKA ET AL: "Measurement of rigidity in Parkinson's disease", MOVEMENT DISORDERS, vol. 12, no. 1, 1 January 1997 (1997-01-01), pages 24-32, XP055099858, ISSN: 0885-3185, DOI: 10.1002/mds.870120106
- GIVEN J D ET AL: "Joint dependent passive stiffness in paretic and contralateral limbs of spastic patients with hemiparetic stroke", JOURNAL OF NEUROLOGY NEUROSURGERY AND PSYCHIATRY, vol. 59, no. 3, 1995, pages 271-279, XP002721833, ISSN: 0022-3050
- HOMBERG V ET AL: "Muscle tone in Huntington's disease", JOURNAL OF NEUROLOGICAL SCIENCES, vol. 121, no. 2, 1 February 1994 (1994-02-01), pages 147-154, XP024345707, ELSEVIER SCIENTIFIC PUBLISHING CO, AMSTERDAM, NL ISSN: 0022-510X, DOI: 10.1016/0022-510X(94)90343-3 [retrieved on 1994-02-01]
- XIA R ET AL: "A comparison of the effects of imposed extension and flexion movements on Parkinsonian rigidity", CLINICAL NEUROPHYSIOLOGY, vol. 117, no. 10, 1 October 2006 (2006-10-01), pages 2302-2307, XP028040214, ELSEVIER SCIENCE, IE ISSN: 1388-2457, DOI: 10.1016/J.CLINPH.2006.06.176 [retrieved on 2006-10-01]
- CHLEBOUN G S ET AL: "The relationship between elbow flexor volume and angular stiffness at the elbow", CLINICAL BIOMECHANICS, vol. 12, no. 6, 1 September 1997 (1997-09-01), pages 383-392, XP004096120, BUTTERWORTH SCIENTIFIC LTD, GUILDFORD, GB ISSN: 0268-0033, DOI: 10.1016/S0268-0033(97)00027-2
- TAKAYUKI ENDO ET AL: "A novel method for systematic analysis of rigidity in Parkinson's disease", MOVEMENT DISORDERS, vol. 24, no. 15, 15 November 2009 (2009-11-15), pages 2218-2224, XP055099856, ISSN: 0885-3185, DOI: 10.1002/mds.22752

## Description

### Technical Field

The present invention relates to a muscle tonus measuring apparatus for measuring muscle tonus characteristics of Parkinson's disease patients and stroke patients, and objectively evaluating the muscle tonus characteristics.

### Background Art

Abnormalities of muscle tonus caused by abnormal stretch reflex are classified into spasticity and rigidity: the former is a pyramidal tract sign and the latter is an extrapyramidal sign. To detect the degree of those is quite important neurological examination. However, it is difficult to evaluate their degree accurately unless a neurologist is experienced. Although the Modified Ashworth Scale for spasticity and the UPDRS (Unified Parkinson Disease Rating Scale) for muscle rigidity are known as evaluation indices that currently are used often in clinical trial, the criteria of both of them are semi-quantitative, and thus a difference between evaluators and a difference by single evaluator may be generated. Accordingly, development of quantitative measuring devices for these is desired.

Many of such measuring devices are for measuring the joint angle of a wrist or an elbow and a torque around them. However, any of such devices requires a large-scale apparatus, and has a drawback in the fit of the apparatus to a subject, which leads to problems such as imposition of a burden on the subject, and consequently there is almost no such device that has been put in practical use.

Patent Document 1 and Non-Patent Document 1 disclose a mitt-type or cuff-type dynamometer provided with a pair of pressure transducers. An evaluator holds the wrist of a subject in a manner such that the wrist is sandwiched with the pair of pressure transducers, and flexes and extends the subject's elbow. At this time, the pushing and pulling forces applied by the evaluator to the subject's wrist are measured via the pair of pressure transducers. Simultaneously, displacement of the subject's forearm is measured. This dynamometer is comparatively small, and fits better to the subject than a conventional device.

Non-Patent Document Given, J.D., et al., "Joint dependent passive stiffness in paretic and contralateral limbs of spastic patients with hemiparetic stroke", Journal of Neurology, 1995, 59, pp. 271-279, discloses a muscle tonus apparatus according to the preamble of claim 1.

### Prior Art Documents

### Patent Document

Patent Document 1: JP H10-511866A

### Non-Patent Document

Non-Patent Document 1: Prochazka, A., et al., "Measurement of rigidity in Parkinson's disease," Movement Disorder Society, 1997.12(1): p.p. 24-32

### Disclosure of Invention

### Problem to be Solved by the Invention

However, data obtained from the dynamometers disclosed in Patent Document 1 and Non-Patent Document 1 above have a low correlation with clinical assessment scores, and thus an abnormality of muscle tonus cannot be evaluated objectively.

An object of the present invention is to solve the above conventional problems, and provide a muscle tonus measuring apparatus that can evaluate muscle tonus easily and objectively with a simple configuration.

### Means for Solving Problem

The object of the invention is solved by a muscle tonus measuring apparatus according to the independent claim.

A muscle tonus measuring apparatus of the present invention is provided with at least one force sensor for detecting a force applied to cause each of flexion and extension movements of a joint of a subject, and a sensor for measuring a joint angle of the joint of the subject. A joint torque for flexing and extending the joint is calculated based on the forces detected by the force sensor. Then, a change with passage of time in each of the joint torque and the joint angle during flexion and extension movements of the joint of the subject is separated into a maximal extension position static phase, a dynamic flexion phase, a maximal flexion position static phase and a dynamic extension phase, and a muscle tonus feature amount is extracted from a relationship between the joint angle and the joint torque in at least one of the dynamic flexion phase and the dynamic extension phase.

### Effects of the Invention

With the present invention, a muscle tonus feature amount is extracted from the relationship between the joint angle and the joint torque in at least one of the joint dynamic flexion phase and the joint dynamic extension phase. By using this muscle tonus feature amount, muscle tonus can be evaluated objectively with a method close to the medical examination manipulation that is performed conventionally by neurologists in medical examinations with an extremely simple apparatus. Accordingly, an objective evaluation index can be provided with respect to a clinical test of a neuromuscular disease, and thus not only an increase in efficiency of clinical tests and reduction in the cost therefor can be achieved, but also clinical practice of neurology can be improved. Furthermore the possibility of elucidation and control of the pathological condition of neuromuscular diseases can be raised by continuously developing kinetic analysis as a science, for instance. Accordingly, the present invention can contribute greatly to an improvement in medical care.

### Brief Description of Drawings

[FIG. 1] FIG. 1 is a diagram showing a schematic configuration of a muscle tonus measuring apparatus according to an embodiment of the present invention.
[FIG. 2] FIG. 2 is a diagram showing a data analysis technique in the muscle tonus measuring apparatus according to the embodiment of the present invention.
[FIG. 3A] FIG. 3A is a typical raw waveform diagram of muscle tonus measurement results from healthy subject.
[FIG. 3B] FIG. 3B is an angle-torque characteristics diagram in a dynamic flexion phase and a dynamic extension phase obtained based on the raw waveform diagram in FIG. 3A.
[FIG. 4A] FIG. 4A is a typical raw waveform diagram of muscle tonus measurement results from Parkinson's disease patient (UPDRS 1).
[FIG. 4B] FIG. 4B is an angle-torque characteristics diagram in the dynamic flexion phase and the dynamic extension phase obtained based on the raw waveform diagram in FIG. 4A.
[FIG. 5A] FIG. 5A is a typical raw waveform diagram of muscle tonus measurement results from Parkinson's disease patient (UPDRS 2).
[FIG. 5B] FIG. 5B is an angle-torque characteristics diagram in the dynamic flexion phase and the dynamic extension phase obtained based on the raw waveform diagram in FIG. 5A.
[FIG. 6A] FIG. 6A is a typical raw waveform diagram of muscle tonus measurement results from Parkinson's disease patient (UPDRS 3).
[FIG. 6B] FIG. 6B is an angle-torque characteristics diagram in the dynamic flexion phase and the dynamic extension phase obtained based on the raw waveform diagram in FIG. 6A.
[FIG. 7A] FIG. 7A is a box plot showing elastic coefficients in dynamic extension phases extracted from data obtained in an example and sorted according to UPDRS rigidity score.
[FIG. 7B] FIG. 7B is a box plot showing elastic coefficients in dynamic flexion phases extracted from data obtained in the example and sorted according to UPDRS rigidity score.
[FIG. 7C] FIG. 7C is a box plot showing sums of bias differences extracted from data obtained in the example and sorted according to UPDRS rigidity score.
[FIG. 7D] FIG. 7D is a box plot showing IEMG ratios of a biceps brachii muscle extracted from data obtained in the example and sorted according to UPDRS rigidity score.
[FIG. 7E] FIG. 7E is a box plot showing IEMG ratios of a triceps brachii muscle extracted from data obtained in the example and sorted according to UPDRS rigidity score.
[FIG. 8] FIG. 8 is a box plot showing IEMG ratios of the biceps brachii muscle obtained in the example and sorted into a healthy subject group and a Parkinson's disease patient group.
[FIG. 9] FIG. 9 is a diagram showing changes with passage of time in torque differentials obtained by time differentiation on elbow joint torques in the dynamic extension phase with regard to a healthy subject, a Parkinson's disease patient without tremor, and a Parkinson's disease patient with tremor.

### Description of the Invention

The above muscle tonus measuring apparatus of the present invention is provided with at least one force sensor. A force applied to flex a joint of a subject and a force applied to extend the joint may be detected by a single common force sensor, or may be detected by respective force sensors.

It is preferable that the at least one force sensor and the sensor for measuring the joint angle are mounted on a common base. Accordingly, the apparatus can be reduced in size, thereby improving the fit to the subject. Further, since the number of components of the apparatus decreases, the setup when performing muscle tonus measurement is easier.

It is preferable that the sensor for measuring the joint angle includes a gyroscope. In this case, the gyroscope detects the angular velocity generated following flexion and extension movements of the joint, and can calculate a joint angle by integrating the detected angular velocity. Accordingly, a joint angle can be measured accurately with a simple and small apparatus.

It is preferable that the muscle tonus feature amount includes an elastic coefficient of the joint. Since an elastic coefficient of a joint has a correlation with the degree of muscle rigidity, the reliability of muscle tonus evaluation improves by using the elastic coefficient.

It is preferable that the elastic coefficient is an elastic coefficient in each of a joint dynamic flexion phase and a joint dynamic extension phase. Accordingly, the reliability of muscle tonus evaluation further improves.

It is preferable that the muscle tonus feature amount includes a feature amount based on a difference between the relationship between the joint angle and the joint torque in the joint dynamic flexion phase and the relationship between the joint angle and the joint torque in the joint dynamic extension phase. Generally, the higher the degree of muscle rigidity becomes, the greater the difference is in the dynamics of a motor system between the dynamic flexion phase and the dynamic extension phase. Thus, according to this preferable aspect, the reliability of muscle tonus evaluation improves.

It is preferable that the muscle tonus measuring apparatus of the present invention further is provided with a surface electrode for measuring a surface myoelectric potential. In this case, it is preferable that a myoelectric activity feature amount further is extracted from the surface myoelectric potential in each of a joint maximal flexion position static phase and a joint maximal extension position static phase. The presence or absence of muscle rigidity has a correlation with a change in the surface myoelectric potentials in the joint maximal flexion position static phase and the joint maximal extension position static phase. Thus, according to this preferable aspect, it is possible to distinguish between a healthy subject and a Parkinson's disease patient with higher accuracy. Further, it is also possible to discriminate the severity of muscle rigidity of a Parkinson's disease patient using the myoelectric activity feature amount.

It is preferable that the muscle tonus measuring apparatus of the present invention further extracts a joint torque feature amount by performing frequency analysis on the joint torque. Accordingly, it is possible to distinguish objectively between a healthy subject and a Parkinson's disease patient and determine whether or not a Parkinson's disease patient has a tremor.

It is preferable that the joint is an elbow joint. Accordingly, muscle tonus can be evaluated objectively with a method closer to the medical examination manipulation that is conventionally performed by neurologists in medical examinations.

Below, the present invention will be described in detail, while disclosing an embodiment. However, it goes without saying that the present invention is not limited to the following embodiment.

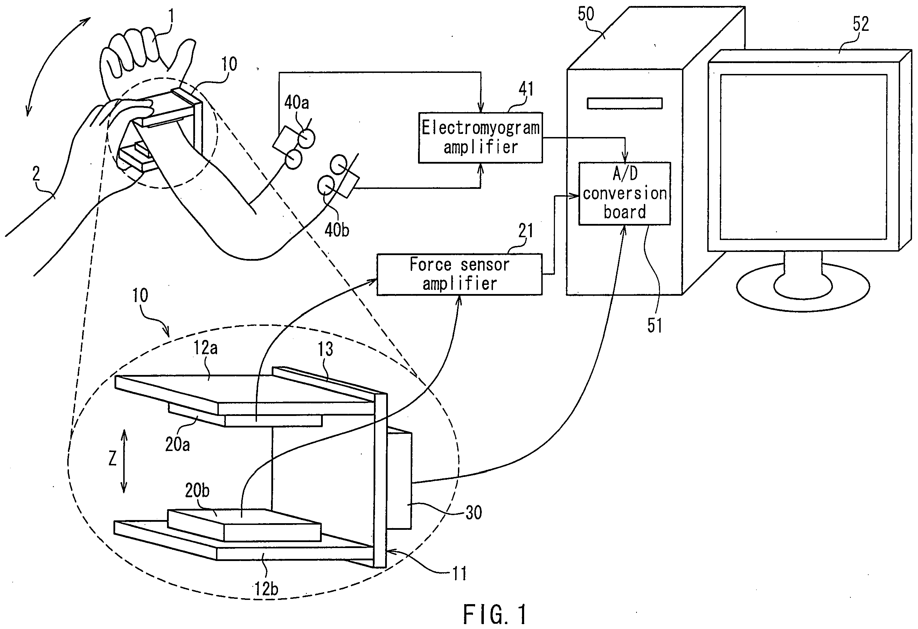

FIG. 1 shows the schematic configuration of a muscle tonus measuring apparatus for measuring muscle tonus characteristics through flexion and extension movements of an elbow joint of a patient according to an embodiment of the present invention.

This muscle tonus measuring apparatus is provided with a detection unit 10, an arithmetic apparatus 50 for performing arithmetic processing on measured data, and an output apparatus 52 for outputting an arithmetic result. The detection unit 10 includes a base 11 that has a substantially square-cornered U shape or a substantially U shape and can be considered as a substantially rigid body. As shown in FIG. 1, the detection unit 10 is fitted around so as to sandwich the wrist joint portion of a subject 1, and an examiner 2 flexes and extends the elbow joint of the subject 1 via the detection unit 10.

A pair of force sensors 20a and 20b are fixed in opposition to each other on a pair of sandwich plates 12a and 12b of the base 11 in opposition to each other. The structure of the force sensors 20a and 20b is not limited as long as a compressive force applied to the force sensors 20a and 20b can be detected, and widely-used force sensors that are conventionally known can be used as the force sensors 20a and 20b. If the direction in which the pair of force sensors 20a and 20b are in opposition is the Z-axis, the force sensors 20a and 20b detect at least a force in the Z-axis direction. For example, widely-used small triaxial force sensors that detect forces in triaxial directions including the Z-axis can be used as the force sensors 20a and 20b. By detecting forces in the orthogonal biaxial directions orthogonal to the Z axis direction in addition to the force in the Z axis direction, the direction of the force applied by the examiner 2 to the subject 1 can be revised, and data of the detected force in the Z axis direction can be corrected. In order to alleviate discomfort due to the force sensors 20a and 20b being in direct contact with the skin of the subject 1 when causing flexion and extension movements of the elbow joint, soft pads may be attached to the faces in opposition to each other (the faces brought into contact with the wrist joint portion of the subject 1) of the force sensors 20a and 20b.

A gyroscope 30 is fixed on a bridge plate 13 that connects the pair of sandwich plates 12a and 12b of the base 11. The gyroscope 30 detects a change in an orientation of the detection unit 10 including the gyroscope 30 that changes following flexion and extension movements of the elbow joint of the subject 1.

The muscle tonus measuring apparatus according to the present embodiment further is provided with surface electrodes 40a and 40b for detecting a surface myoelectric potential. The surface electrodes 40a and 40b are respectively attached to the positions of the biceps brachii muscle and the triceps brachii muscle of the subject 1. Known electrodes used for measuring the surface myoelectric potential can be utilized as the surface electrodes 40a and 40b.

When flexion and extension movements of the elbow joint of the subject 1 are caused, the detection unit 10 moves along the circular arc with the elbow joint serving as the center. During flexion and extension movements of the elbow joint, the orientation of the detection unit 10 is maintained such that the Z-axis is always parallel to the tangential direction of this circular arc. The force sensors 20a and 20b output a voltage according to the force in the Z axis direction applied by the examiner 2 to the subject 1 when the examiner 2 causes flexion and extension movements of the elbow joint of the subject 1. The voltage output from the force sensors 20a and 20b is amplified by a force sensor amplifier 21 as necessary, and thereafter is input to the arithmetic apparatus 50 via an A/D conversion board 51. A voltage output from the gyroscope 30 according to a change in the orientation thereof is input to the arithmetic apparatus 50 via the A/D conversion board 51. Voltages output from the surface electrodes 40a and 40b are amplified by an electromyogram amplifier 41 as necessary, and thereafter input to the arithmetic apparatus 50 via the A/D conversion board 51.

A joint torque, a joint angle, and surface myoelectric potentials are measured while repeating, with respect to the elbow joint of the subject 1, four phases, namely, (1) a maximal extension position static phase, (2) a dynamic flexion phase, (3) a maximal flexion position static phase, and (4) a dynamic extension phase.

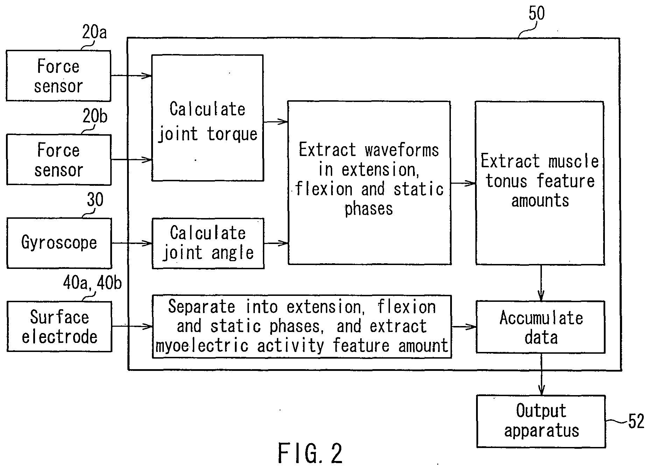

FIG. 2 is a diagram showing a technique of data analysis performed by the arithmetic apparatus 50 in the muscle tonus measuring apparatus according to the present embodiment.

A joint torque is calculated based on the force in the Z axis direction detected via the force sensors 20a and 20b, and the distance between the elbow joint of the subject 1 and the position where the detection unit 10 is fitted around, which has been measured separately (that is, a radius of the circular arc along which the detection unit 10 moves in the flexion and extension movements). A joint angle is calculated by integrating a change in the orientation (angular velocity) of the detection unit 10 detected via the gyroscope 30. Then, the change with passage of time (raw waveform) in each of the joint torque and the joint angle is separated into the above four phases, and muscle tonus feature amounts are extracted from the relationship between the joint angle and the joint torque in each of the dynamic flexion phase and the dynamic extension phase. Examples of the muscle tonus feature amounts include an elastic coefficient in a joint dynamic flexion phase, an elastic coefficient in a joint dynamic extension phase, and the sum of bias differences (details will be described later).

Furthermore, the change with passage of time (raw waveform) in the surface myoelectric potentials detected via the surface electrodes 40a and 40b is separated into the above four phases, and a myoelectric activity feature amount is extracted. An example of the myoelectric activity feature amount is an IEMG ratio (the details will be described later).

Further, although not shown, a joint torque feature amount may be extracted by performing frequency analysis on the change with passage of time in a joint torque in the dynamic extension phase and/or the dynamic flexion phase.

Data with regard to the above-described muscle tonus feature amounts, myoelectric activity feature amount and joint torque feature amount obtained by the arithmetic apparatus 50 may be accumulated in the arithmetic apparatus 50. Further, the arithmetic apparatus 50 may determine the severity of muscle tonus by analyzing the accumulated data with a statistical technique, for example. Raw waveform diagrams of a joint angle, a joint torque, and a surface myoelectric potential, various feature amounts described above, and further the severity determination result, for instance, are output to the output apparatus 52 in response to a request from the examiner. For example, a widely-used personal computer can be used as the arithmetic apparatus 50. For example, any of various displays and printers can be used as the output apparatus 52.

The muscle tonus measuring apparatus achieved by the present invention is a revolutionary system that imposes less burden on the patient due to the detection unit 10 fitting around, and can quantify muscle tonus with a method as close as possible to the medical examination manipulation that is performed conventionally by neurologists in routine medical examinations. Particularly, there is no other example in the world until now as a technique of dividing flexion and extension movements into two dynamic phases in which continuous flexion and extension is performed and two static phases at a maximal flexion position and a maximal extension position, and extracting a feature amount from each phase.

Although the muscle tonus measuring apparatus provided with the surface electrodes 40a and 40b has been described in the above embodiment, the surface electrodes 40a and 40b can be omitted in the present invention. As shown in an example described later, the severity of muscle rigidity of a Parkinson's disease patient can be discriminated objectively by using muscle tonus feature amounts obtained from the relationship between a joint angle and joint torque in each of the dynamic flexion phase and the dynamic extension phase.

Moreover, it is possible to distinguish objectively between a healthy subject (UPDRS 0) and a Parkinson's disease patient having identified muscle rigidity (UPDRS 1 or more) by detecting the surface myoelectric potentials using the surface electrodes. Further, the severity of muscle rigidity of a Parkinson's disease patient also can be discriminated objectively.

With the conventional technique disclosed in Patent Document 1 and Non-Patent Document 1 described above, the elbow joint is caused to perform sinusoidal flexion and extension movements at 1 Hz or less, and a mechanical impedance, which is the vectorial sum of the elasticity (elastic coefficient) of the elbow joint and the viscosity thereof, is calculated, assuming that the motor system around the elbow joint at this time is a time-invariant linear system. However, as is clear from the example described later, the dynamics of a motor system in the dynamic flexion phase and those in the dynamic extension phase are not actually the same. With the above conventional technique, this point was overlooked, which is considered to be the major cause of the difference between obtained data and clinical assessment. In contrast, with the measuring apparatus of the present invention, the dynamic flexion phase and the dynamic extension phase are considered to be different motor systems, and a plurality of feature amounts (muscle tonus feature amounts) including an elastic coefficient are extracted from the relationship between a joint angle and a joint torque in each of the phases. This enables objective evaluation having an extremely high correlation with clinical assessment to be performed with regard to muscle rigidity of a Parkinson's disease patient. Moreover, a healthy subject and a Parkinson's disease patient also can be distinguished from each other with the addition of the feature amount (myoelectric activity feature amount) with regard to the myoelectric activities in the static maximal extension phase and the static maximal flexion phase. Further, it is possible to determine whether or not a Parkinson's disease patient has a tremor and to distinguish between a healthy subject and a Parkinson's disease patient, using the joint torque feature amount. Thus, the present invention enables discrimination of the severity of abnormalities of muscle tonus and the pattern classification according to the pathological condition, and thus extremely useful information can be provided in a routine clinical practice by neurologists.

Further, in Patent Document 1 and Non-Patent Document 1 described above, the forces applied when causing flexion and extension movements of a joint of a subject are detected via pressure pads filled with air (or fluid). The responsiveness of force detection is poor with this technique. In contrast, a force sensor is used in the present invention. Since responsiveness of a force sensor is generally favorable, a joint torque can be measured with high accuracy in real time. Further, since a force sensor is generally small and has a light weight, the burden on a subject due to the detection unit 10 fitting around can be reduced.

The present invention is not limited to the above embodiment nor to the following example, and various modifications can be made.

For example, the shape of the base where the force sensors 20a and 20b and the gyroscope 30 are mounted does not need to be a substantially square-cornered U shape or a substantially U shape as in the above embodiment, and for example, the whole shape thereof may be any of a circle, an ellipse and various polygons including a quadrangle, for instance, and may be a toroidal shape with a through-hole in the center. Furthermore, for the purpose of improving the fit to a subject, the base may have a movable portion or a part of the base or the entire base may have flexibility.

Instead of the pair of force sensors 20a and 20b, a single force sensor for detecting pushing and pulling forces applied when the examiner 2 causes flexion and extension movements of the elbow joint of the subject 1 may be used.

A stage for supporting the subject's joint may be provided, and a distance sensor for automatically measuring the distance between this stage and the detection unit 10 further may be provided. Accordingly, the turning radius of the detection unit 10 necessary for calculating a joint torque can be measured easily.

In the above embodiment, the gyroscope 30 for measuring a joint angle is mounted on the base 11 together with the force sensors 20a and 20b. Accordingly, the entire apparatus can be reduced in size, and a joint torque and a joint angle can be simultaneously measured by only fitting the detection unit 10 around the subject. However, in the present invention, the method for measuring a joint angle is not limited to this, and a known angular change measurement method can be utilized. For example, other than the detection unit 10 including the force sensors 20a and 20b, a sensor for angle measurements (for example, a potentiometer or a rotary encoder) may be fitted in the vicinity of a joint of the subject via a jig.

The arithmetic apparatus for performing predetermined arithmetic processing using measured data and the display apparatus for displaying an arithmetic result may be reduced in size and mounted on the detection unit 10.

It is of course possible to apply the measuring apparatus of the present invention to joints other than the elbow joint. According to the joint to be applied, the shape of the detection unit 10, the points where the surface electrodes are attached, and the like can be changed appropriately.

### Examples

With respect to 27 patients diagnosed as having Parkinson's disease based on Parkinson's disease diagnostic criteria (1995) by the research study group of specified diseases and neurodegenerative diseases of the Ministry of Health and Welfare (16 men and 11 women, aged 47 to 85) and 24 healthy subjects (18 men and 6 women, aged 55 to 85), muscle tonus characteristics were measured using the muscle tonus measuring apparatus shown in FIG.1.

Prior to the measurement, the healthy subjects underwent neurological examination, and the Parkinson's disease patients were assessed using UPDRS(Unified Parkinson Disease Rating Scale) Part III, and muscle rigidity was given a score in 5 levels from 0 to 4 (see Table 1).

**Table 1**

| Muscle rigidity scores (Patient at rest. Determine based on main joints. Cogwheel phenomenon not to be recorded.) | |
|---|---|
| 0: | Absent. |
| 1: | Slight or degree that mirror or other movements may induce. |
| 2: | Mild to moderate muscle rigidity. |
| 3: | Marked muscle rigidity, but range of joint motion is normal. |
| 4: | Severe muscle rigidity, and range of joint motion is limited. |

Subjects were instructed to remain relaxed in a rest sitting position, and the examiner supported the elbow joint portion of the subject with one hand, and caused passive flexion and extension movements of the elbow joint of the subject, holding the wrist joint portion of the subject with the other hand via the detection unit 10. The measurement started from a maximal extension position, and a repeating unit defined by remaining still for 3 seconds at a maximal extension position, flexing taking 2 seconds, remaining still for 3 seconds at a maximal flexion position, and extending taking 2 seconds was repeated for 60 seconds. This measurement was conducted twice for each of the left and right upper limbs with respect to one subject, the reproducibility was confirmed, and one data piece each for the left and right (in total, two data pieces) was obtained per one subject. Among all the obtained data, data with regard to a healthy subject who had clinically identified muscle rigidity was excluded, and also data considered to include obvious voluntary movement by visual observation of the electromyogram, data considered to include noise due to malfunction of the measuring apparatus, and the like were excluded. Finally, 39 data pieces with regard to the healthy subjects and 50 data pieces with regard to the Parkinson's disease patients, that is, 89 data pieces in total, were obtained.

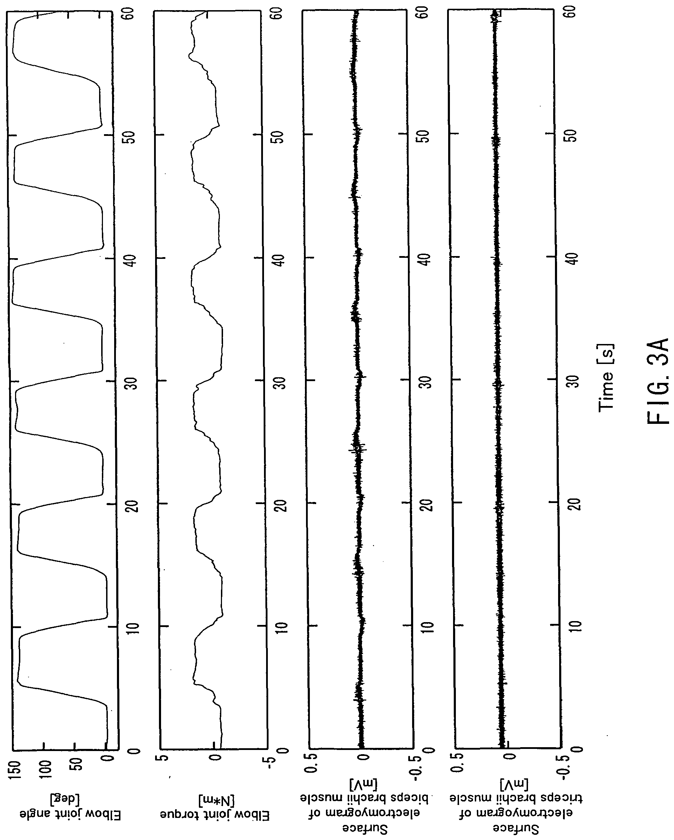

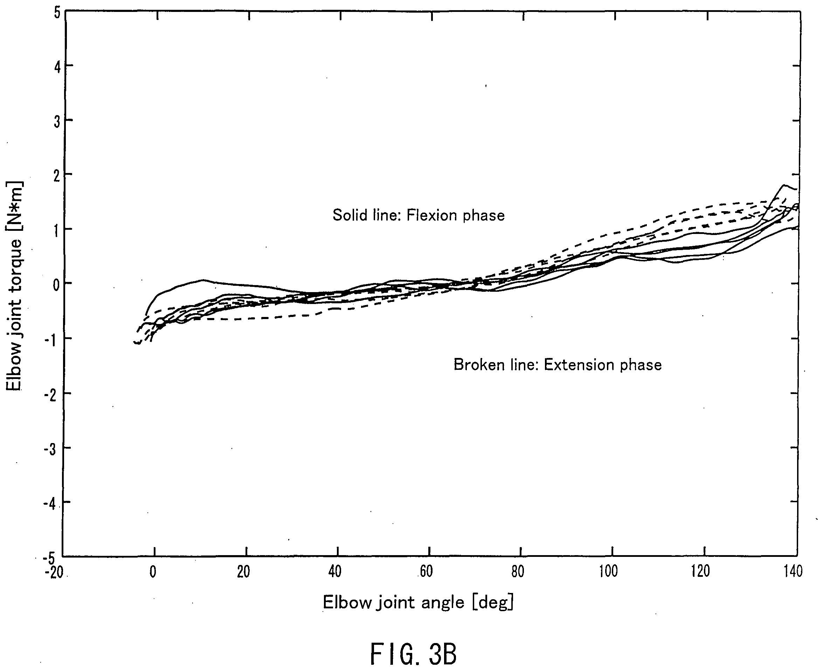

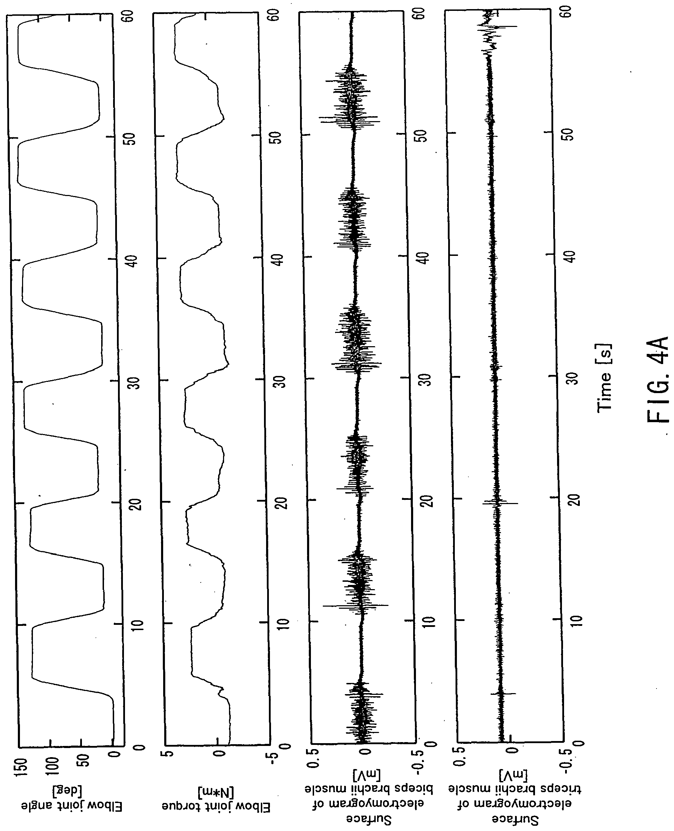

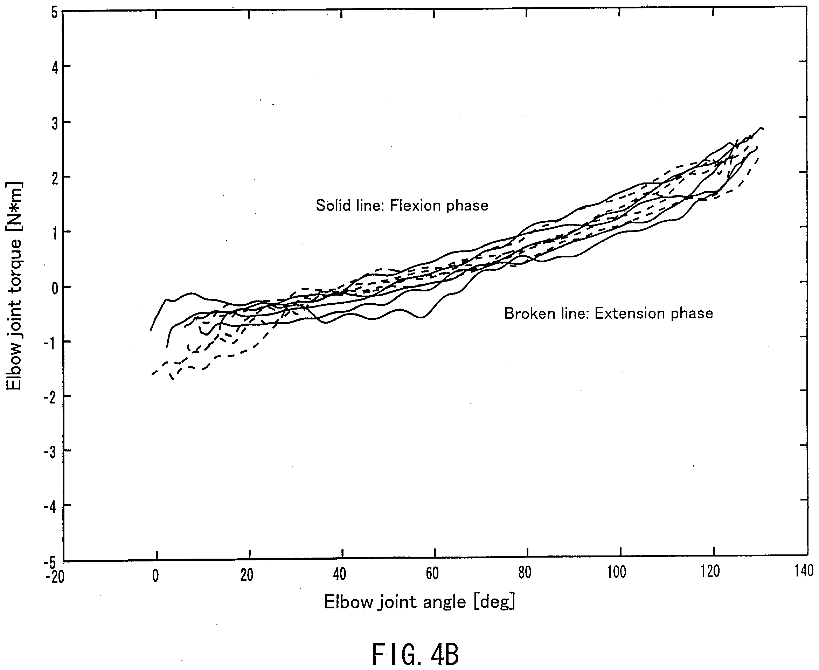

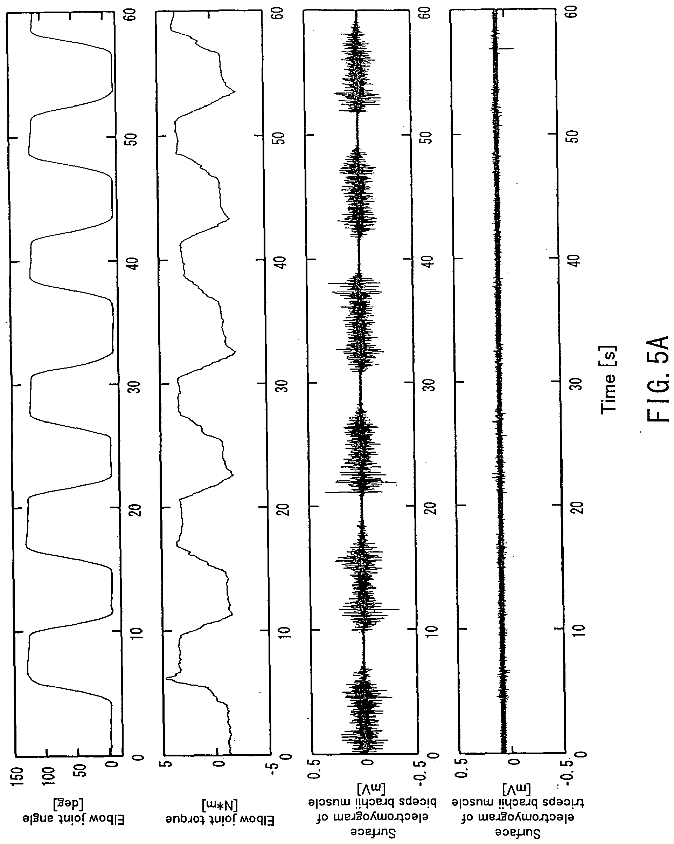

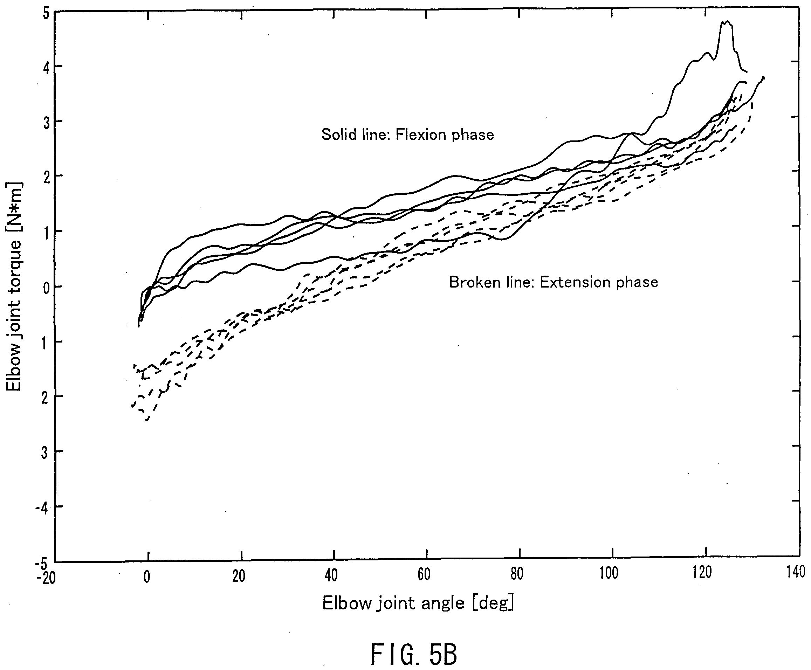

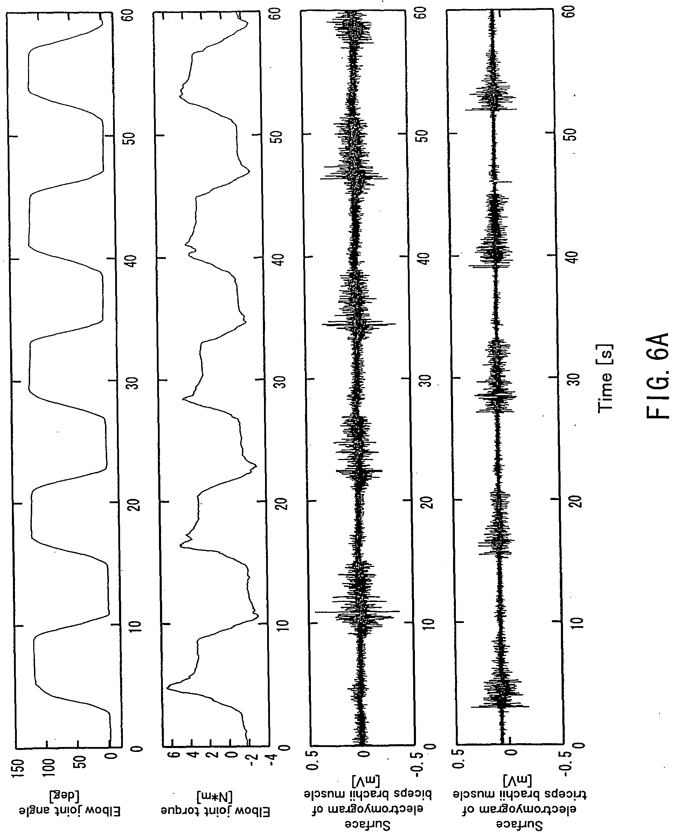

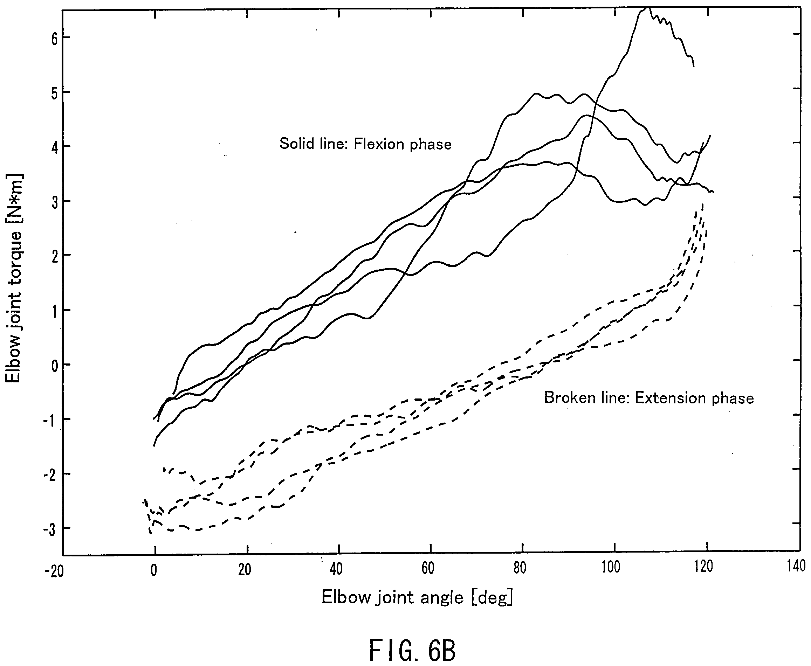

Raw waveform diagrams of typical measurement results from healthy subject and Parkinson's disease patient (UPDRS 1 to 3) are shown in FIGS. 3A, 4A, 5A, and 6A. Further, angle-torque characteristics diagrams in the dynamic flexion phase and the dynamic extension phase, which are obtained based on the raw waveform diagrams, are shown in FIGS. 3B, 4B, 5B, and 6B.

An elastic coefficient of an elbow joint in a dynamic flexion phase, an elastic coefficient of an elbow joint in a dynamic extension phase and the sum of bias differences as muscle tonus feature amounts, and further an IEMG ratio as a myoelectric activity feature amount were each extracted from the measured data. Methods for extracting these are as follows.

### Elastic coefficient

In each of the angle-torque characteristics diagrams, the range where a joint angle is 10° to 110° was extracted from among all the data points in the flexion phase of all the performed measurements, and a regression line was obtained. The slope of the regression line was defined as an elastic coefficient in the dynamic flexion phase. An elastic coefficient in the dynamic extension phase was defined in the same manner.

### Sum of bias differences

In each of the angle-torque characteristics diagrams, the mean value of torques obtained by all the performed measurements in the flexion phase at one certain joint angle was defined as the bias in the dynamic flexion phase. The bias in the dynamic extension phase was defined in the same manner. The difference between the bias in the dynamic flexion phase and the bias in the dynamic extension phase was defined as a bias difference. A bias difference was calculated with respect to three joint angles, 30°, 60° and 90°, and the sum of the differences was defined as the sum of bias differences.

### IEMG ratio

A surface electromyogram waveform in each of the maximal flexion phase (when being still at a maximal flexion position) and the maximal extension phase (when being still at a maximal extension position) was rectified and smoothed using a 2.5-Hz low pass filter. Subsequently, an integrated value of the rectified and smoothed electromyogram waveform (Integrated EMG = IEMG) for one second was calculated. The IEMG ratio of the biceps brachii muscle was defined as follows: (IEMG ratio of the biceps brachii muscle) = (IEMG in the maximal extension phase)/(IEMG in the maximal flexion phase). The IEMG ratio of the triceps brachii muscle was defined as follows: (IEMG ratio of the triceps brachii muscle) = (IEMG in the maximal flexion phase)/(IEMG in the maximal extension phase). The IEMG ratio is a feature amount indicating the strength of a myoelectric activity in the state where a muscle is passively extended. The IEMG ratio was calculated for each of the biceps brachii muscle and the triceps brachii muscle.

Since it was considered that these feature amounts such as elastic coefficients, the sum of bias differences, and IEMG ratios depended on the muscle mass of each subject, the feature amounts were normalized using the mass of the brachium portion estimated based on the subject's weight. Moreover, prior to the feature amount extraction, with respect to a joint torque, a torque due to gravity was corrected.

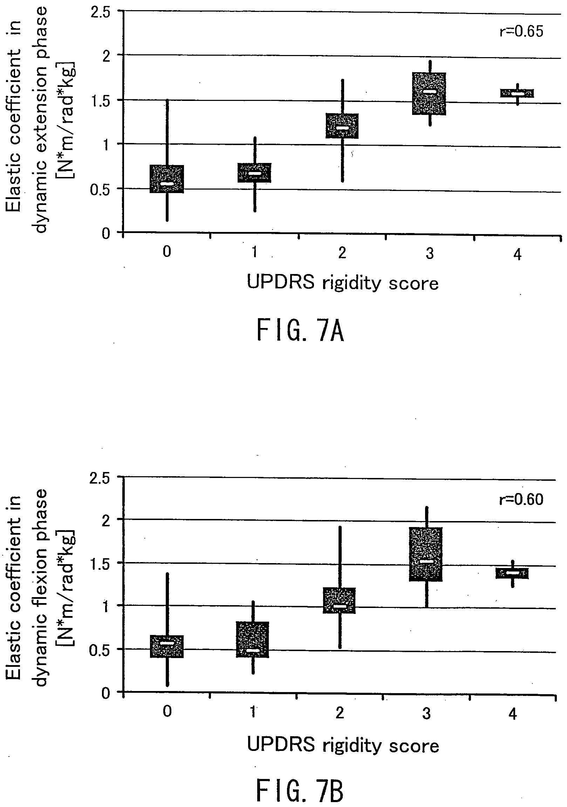

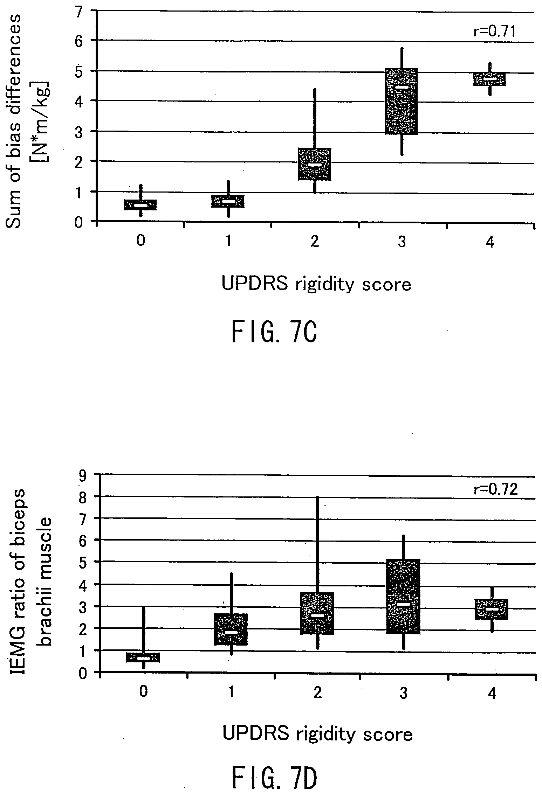

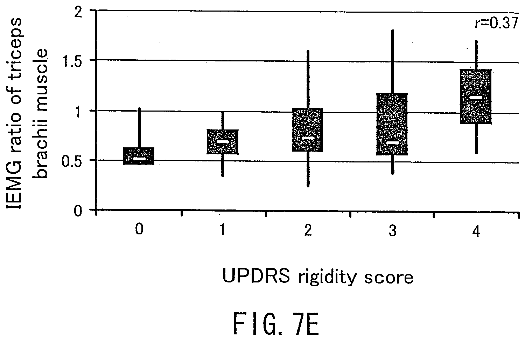

FIGS. 7A to 7E are box plots showing elastic coefficients in dynamic extension phases, elastic coefficients in dynamic flexion phases, the sums of bias difference, IEMG ratios of a biceps brachii muscle, and IEMG ratios of a triceps brachii muscle that were extracted from the 89 data pieces described above and sorted according to UPDRS rigidity score. Coefficients r of correlation with UPDRS are 0.65 for the elastic coefficient in the dynamic extension phase (95% CI (confidence interval): 0.51 to 0.75), 0.60 for the elastic coefficient in the dynamic flexion phase (95% CI: 0.45 to 0.72), 0.71 for the sum of bias difference (95% CI: 0.59 to 0.80), 0.72 for the IEMG ratio of the biceps brachii muscle (95% CI: 0.60 to 0.81), and 0.37 for the IEMG ratio of the triceps brachii muscle (95% CI: 0.17 to 0.54), and all of these show a correlation with UPDRS. Accordingly, it was considered that the severity of muscle rigidity of a Parkinson's disease patient can be discriminated by using at least one of the elastic coefficient in the dynamic extension phase, the elastic coefficient in the dynamic flexion phase, the sum of bias differences, the IEMG ratio of the biceps brachii muscle, and the IEMG ratios of the triceps brachii muscle. Further, it was considered that a combination of two or more of these feature amounts improves the reliability of discrimination of the severity of muscle rigidity of a Parkinson's disease patient.

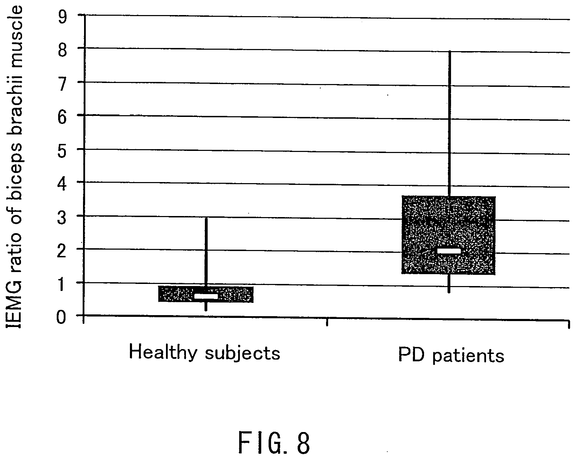

FIG. 8 is a box plot showing the IEMG ratios of the biceps brachii muscle, which have been sorted into a healthy subject group and a Parkinson's disease (PD) patient group. Among 39 data pieces with regard to healthy subjects, 31 data pieces show that the IEMG ratio of the biceps brachii muscle is less than 1.1, whereas among 50 data pieces with regard to PD patients, 46 data pieces show that the ratio is 1.1 or more, and thus it was considered that PD patients were in the state where muscle tone was significantly facilitated. The IEMG ratio of the biceps brachii muscle has the sensitivity of 92% and the specificity of 80% when a cutoff value is set to 1.1, and thus was considered to be useful to distinguish between a healthy subject and a PD patient.

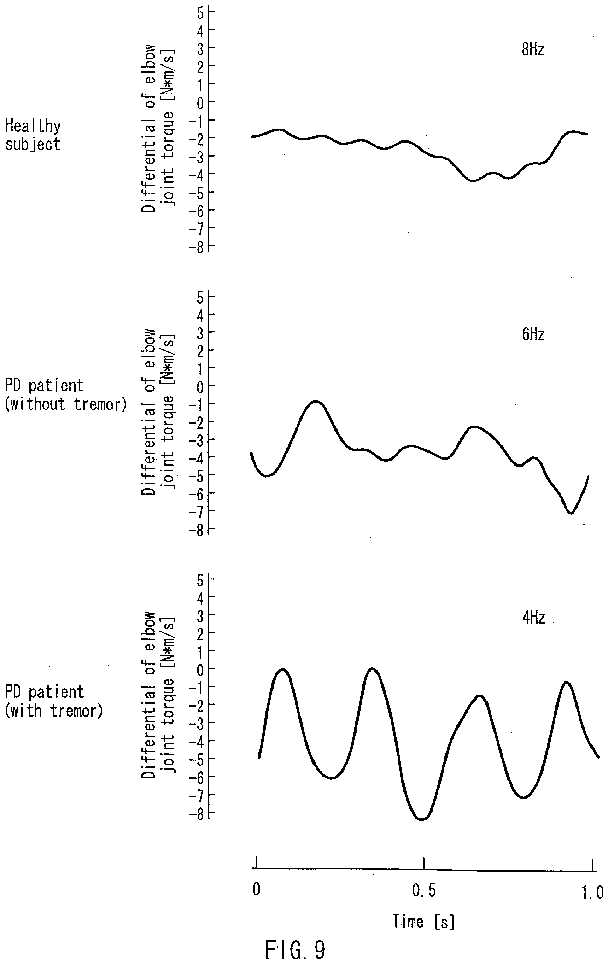

FIG. 9 is a diagram showing changes with passage of time in torque differential values obtained by time differentiation on elbow joint torques in the dynamic extension phase with regard to a healthy subject, a PD patient without tremor, and a PD patient with tremor in this order from top. With a healthy subject, a low amplitude torque differential value at about 8 Hz was observed, and with a PD patient without tremor, an irregular amplitude torque differential value at about 6 Hz was observed. With a PD patient with tremor, a high amplitude torque differential value at about 4 Hz was observed. The frequency of macroscopic tremor of PD patient with tremor was about 4 Hz, and it was considered that this was able to have been extracted. In contrast, it was considered that the feature component for a healthy subject is an about 8-Hz component and that for a PD patient without tremor is an about 6-Hz component. Thus, the peak frequency of a joint torque can be extracted as a joint torque feature amount by performing frequency analysis on the joint torque, and it was considered that, using this feature amount, it is possible to distinguish objectively between a healthy subject and a PD patient and determine whether or not a PD patient has a tremor.

In the above example, the elastic coefficient was obtained based on the slope of the regression line in the range where a joint angle is 10° to 110° in each of the angle-torque characteristics diagrams. However, the range of joint motion differs for each subject, and thus there are cases where it is not appropriate to obtain the elastic coefficient based on the slope of the regression line in this joint angle range. In such cases, the elastic coefficient may be estimated based on the frequency characteristics in an angle-torque system. The inventors of the present invention have confirmed that elastic coefficients calculated with this technique are almost the same as the elastic coefficients calculated based on the angle-torque characteristics diagrams.

Further, although time differential is performed on the elbow-joint torques in the dynamic extension phase in FIG. 9, it has been confirmed that even if time differential is performed on the elbow-joint torques in the dynamic flexion phase, similar joint torque feature amounts as those in FIG. 9 can be extracted, and using these feature amounts, it is possible to objectively distinguish between a healthy subject and a PD patient and determine whether or not a PD patient has a tremor.

### Industrial Applicability

Although the utilization field of the present invention is not particularly limited, the present invention can be utilized over a large range such as determination of the severity of Parkinson's disease, and determination of therapeutic effects before and after antiparkinson drug administration, for example.

### Description of Reference Numerals

- 10: Detection unit
- 11: Base
- 12a and 12b: Sandwich plate
- 13: Bridge plate
- 20a and 20b: Force sensor
- 21: Force sensor amplifier
- 30: Gyroscope
- 40a and 40b: Surface electrode
- 41: Electromyogram amplifier
- 50: Arithmetic apparatus
- 51: A/D conversion board
- 52: Output apparatus

## Claims

1. A muscle tonus measuring apparatus, comprising:
at least one force sensor (20a, b) for detecting a force applied to cause each of flexion and extension movements of a joint of a subject;
a sensor (30) for measuring a joint angle of the joint of the subject, and
a surface electrode (40a, b) for measuring a surface myoelectric potential,
an arithmetic apparatus (50) that is configured to calculate a joint torque for flexing and extending the joint based on the forces detected by the force sensor,
separate a change with passage of time in each of the joint torque and the joint angle during flexion and extension movements of the joint of the subject into a maximal extension position static phase, a dynamic flexion phase, a maximal flexion position static phase, and a dynamic extension phase, and
extract a muscle tonus feature amount from a relationship between the joint angle and the joint torque in at least one of the dynamic flexion phase and the dynamic extension phase,
**characterised in that** the arithmetic apparatus (50) is further configured to extract a myoelectric activity feature amount from the surface myoelectric potential in each of the maximal flexion position static phase and the maximal extension position static phase, the myoelectric activity feature being an integrated EMG (IEMG) value calculated by integrating a rectified and smoothed electromyogram waveform in the respective maximal position static phase for one second.

2. The muscle tonus measuring apparatus according to claim 1, wherein an IEMG ratio is calculated, the IEMG ratio being the ratio of the IEMG value in the maximal extension position static phase to the IEMG value in the maximal flexion position static phase.

3. The muscle tonus measuring apparatus according to claim 1 or 2, wherein an IEMG ratio is calculated, the IEMG ratio being the ratio of the IEMG value in the maximal flexion position static phase to the IEMG value in the maximal extension position static phase.

4. The muscle tonus measuring apparatus according to claim 1, 2 or 3,
wherein the muscle tonus feature amount includes an elastic coefficient of the joint.

5. The muscle tonus measuring apparatus according to claim 4, wherein the elastic coefficient is an elastic coefficient in each of the dynamic flexion phase and the dynamic extension phase.

6. The muscle tonus measuring apparatus according to any one of claims 1 to 5, wherein the muscle tonus feature amount includes a feature amount based on a difference between the relationship between the joint angle and the joint torque in the dynamic flexion phase and the relationship between the joint angle and the joint torque in the dynamic extension phase.

7. The muscle tonus measuring apparatus according to any one of claims 1 to 6, wherein a joint torque feature amount is further extracted by performing frequency analysis on the joint torque.

8. The muscle tonus measuring apparatus according to any one of claims 1 to 7, wherein the joint is an elbow joint.

## Patentansprüche

1. Muskel-Tonus-Messvorrichtung, umfassend:
mindestens einen Kraftsensor (20a, b) zum Nachweis einer zum Hervorrufen jeder von Beuge- und Streck-Bewegungen eines Gelenks einer Person angewendeten Kraft;
einen Sensor (30) zum Messen eines Gelenk-Winkels des Gelenks der Person, und
eine Oberflächenelektrode (40a, b) zum Messen eines myoelektrischen Oberflächenpotenzials,
eine arithmetische Einrichtung (50), ausgelegt zum Berechnen eines Gelenk-Drehmoments zum Beugen und Strecken des Gelenks, basierend auf den durch den Kraftsensor nachgewiesenen Kräften,
Abtrennen einer Änderung im Laufe der Zeit in jedem von dem Gelenk-Drehmoment und dem Gelenk-Winkel während Beuge- und Streck-Bewegungen des Gelenks der Person zu einer maximalen Streck-Position in statischer Phase, einer dynamischen Beuge-Phase, einer maximalen Beuge-Position in statischer Phase, und einer dynamischen Streck-Phase, und
Extrahieren eines Muskel-Tonus-Eigenschaftswerts aus einer Beziehung zwischen dem Gelenk-Winkel und den Gelenk-Drehmoment in mindestens einem von der dynamischen Beuge-Phase und der dynamischen Streck-Phase,
**dadurch gekennzeichnet, dass** die arithmetische Vorrichtung (50) weiterhin zum Extrahieren eines myoelektrischen Aktivitäts-Eigenschaftswerts aus dem myoelektrischen Oberflächenpotenzial in jeder von der maximalen Beuge-Position in statischer Phase und der maximalen Streck Position in statischer Phase ausgelegt ist,
wobei das myoelektrische Aktivitäts-Merkmal ein integrierter EMG (IEMG)-Wert ist, berechnet durch Integrieren einer gleichgerichteten und geglätteten Elektromyogramm-Wellenform in der jeweiligen maximalen Position in statischer Phase für eine Sekunde.

2. Muskel-Tonus-Messvorrichtung nach Anspruch 1, wobei ein IEMG-Verhältnis berechnet wird, wobei das IEMG-Verhältnis das Verhältnis von dem IEMG-Wert in der maximalen Streck Position in statischer Phase zu dem IEMG-Wert in der maximalen Beuge-Position in statischer Phase ist.

3. Muskel-Tonus-Messvorrichtung nach Anspruch 1 oder 2, wobei ein IEMG-Verhältnis berechnet wird, wobei das IEMG-Verhältnis das Verhältnis von dem IEMG-Wert in der maximalen Beuge-Position in statischer Phase zu dem IEMG-Wert in der maximalen Dehnungs-Position in statischer Phase ist.

4. Muskel-Tonus-Messvorrichtung nach Anspruch 1, 2 oder 3, wobei der Muskel-Tonus-Eigenschaftswert einen Elastizitäts-Koeffizient des Gelenks einschließt.

5. Muskel-Tonus-Messvorrichtung nach Anspruch 4, wobei der Elastizitäts-Koeffizient ein Elastizitäts-Koeffizient in jeder von der dynamischen Beuge-Phase und der dynamischen Dehnungs-Phase ist.

6. Muskel-Tonus-Messvorrichtung nach einem der Ansprüche 1 bis 5, wobei der Muskel-Tonus-Eigenschaftswert einen Eigenschaftswert, basierend auf einer Differenz zwischen der Beziehung zwischen dem Gelenk-Winkel und dem Gelenk-Drehmoment in der dynamischen Beuge-Phase und der Beziehung zwischen dem Gelenk-Winkel und dem Gelenk-Drehmoment in der dynamischen Dehnungs-Phase, einschließt.

7. Muskel-Tonus-Messvorrichtung nach einem der Ansprüche 1 bis 6, wobei weiterhin ein Gelenk-Drehmoment-Eigenschaftswert durch Ausführen einer FrequenzAnalyse des Gelenk-Drehmoments extrahiert wird.

8. Muskel-Tonus-Messvorrichtung nach einem der Ansprüche 1 bis 7, wobei das Gelenk ein Ellenbogen-Gelenk ist.

## Revendications

1. Appareil de mesure du tonus musculaire, comprenant :
au moins un capteur (20a, b) de force pour détecter une force qui s'exerce pour déclencher chacun des mouvements de flexion et d'extension d'une articulation d'un sujet ;
un capteur (30) pour mesurer un angle d'articulation de l'articulation du sujet ; et
une électrode (40a, b) de surface pour mesurer un potentiel myoélectrique de surface ;
un appareil arithmétique (50) qui est configuré pour :
calculer un couple d'articulation pour la flexion et l'extension de l'articulation en se basant sur les forces détectées par le capteur de force ;
séparer, un changement qui se manifeste au cours du temps dans respectivement le couple d'articulation et l'angle d'articulation lors des mouvements de flexion et extension de l'articulation du sujet, en une phase statique en position d'extension maximale, une phase de flexion dynamique, une phase statique de position en flexion maximale et une phase d'extension dynamique ; et
extraire une valeur caractéristique du tonus musculaire à partir d'une relation entre l'angle d'articulation et le couple d'articulation dans au moins une phase choisie parmi la phase de flexion dynamique et la phase d'extension dynamique ;
**caractérisé en ce que** l'appareil arithmétique (50) est également configuré pour extraire une valeur caractéristique de l'activité myoélectrique à partir du potentiel myoélectrique de surface respectivement dans la phase statique en position de flexion maximale et dans la phase statique en position d'extension maximale, la caractéristique d'activité myoélectrique représentant une valeur EMG intégrée (IEMG) calculée en intégrant une forme d'onde d'électromyogramme rectifiée et lissée dans la phase statique respective en position maximale, pendant une seconde.

2. Appareil de mesure du tonus musculaire selon la revendication 1, dans lequel un rapport IEMG est calculé, le rapport IEMG représentant le rapport de la valeur IEMG dans la phase statique en position d'extension maximale à la valeur IEMG dans la phase statique en position de flexion maximale.

3. Appareil de mesure du tonus musculaire selon la revendication 1 ou 2, dans lequel un rapport IEMG est calculé, le rapport IEMG représentant le rapport de la valeur IEMG dans la phase statique en position de flexion maximale à la valeur IEMG dans la phase statique en position d'extension maximale.

4. Appareil de mesure du tonus musculaire selon la revendication 1, 2 ou 3, dans lequel la valeur caractéristique du tonus musculaire comprend un coefficient élastique de l'articulation.

5. Appareil de mesure du tonus musculaire selon la revendication 4, dans lequel le coefficient élastique est un coefficient élastique dans respectivement la phase de flexion dynamique et la phase d'extension dynamique.

6. Appareil de mesure du tonus musculaire selon l'une quelconque des revendications 1 à 5, dans lequel la valeur caractéristique du tonus musculaire comprend une valeur caractéristique basée sur une différence entre la relation entre l'angle d'articulation et le couple d'articulation dans la phase de réflexion dynamique et la relation entre l'angle d'articulation et le couple d'articulation dans la phase d'extension dynamique.

7. Appareil de mesure du tonus musculaire selon l'une quelconque des revendications 1 à 6, dans lequel une valeur caractéristique de couple d'articulation est en outre extraite en soumettant le couple d'articulation à une analyse de fréquence.

8. Appareil de mesure du tonus musculaire selon l'une quelconque des revendications 1 à 7, dans lequel l'articulation est l'articulation d'un coude.
